# EUROPEAN PATENT APPLICATION

(11) **EP 4 736 743 A1**
(43) Date of publication of application: **06.05.2026**
(21) Application number: 24845814.3
(22) Date of filing: 19.06.2024
(51) Int. Cl.: A61B 5/00, A61B 5/397, G16H 50/20

(54) **BIOFEEDBACK-BASED BRUXISM ALLEVIATION SYSTEM AND METHOD THEREFOR**

(30) Priority: 24.07.2023 KR 20230096122
(71) Applicant: Miraclare Co., Ltd, Changwon-si Gyeongsangnam-do 51352 (KR)
(72) Inventor: BYUN, Jung Hwan, Changwon-si Gyeongsangnam-do 51106 (KR); KIM, Deok Hyun, Changwon-si Gyeongsangnam-do 51350 (KR)
(74) Representative: BCKIP Part mbB
(86) International application number: PCT/KR2024/008426
(87) International publication number: WO 2025/023510

(57) **Abstract**

A biofeedback-based bruxism alleviating system and a method thereof are disclosed. The biofeedback-based bruxism alleviating system according to one embodiment of the present disclosure includes a user recognition unit that receives a bruxism diagnosis request signal generated from at least one of a predetermined user terminal and a wearable device attached to skins of a user, that extracts a unique number assigned to the wearable device included in the bruxism diagnosis request signal, and that calls up user information corresponding to the unique number from a predetermined DB, a data collection unit that receives electromyography data collected from at least one sensor provided in the wearable device, a bruxism diagnosis unit that diagnoses occurrence of bruxism by determining whether the electromyography data collected from the data collection unit satisfies a preset condition, a biofeedback generation unit that applies a vibration stimulation to the skins of the user through a vibration motor provided in the wearable device, when the bruxism diagnosis unit determines the occurrence of the bruxism, and a user monitoring unit that monitors bruxism symptoms of the user, based on bruxism diagnosis data derived by the bruxism diagnosis unit and vibration stimulation data generated by the biofeedback generation unit.

## Description

### [TECHNICAL FIELD]

The present disclosure relates to a bruxism diagnosis and alleviation system, and more specifically, relates to a biofeedback-based bruxism alleviating system and a method thereof, which can alleviate bruxism symptoms by analyzing a temporal muscle signal of a user who falls asleep, diagnosing the bruxism symptoms, and providing a biofeedback signal including a vibration stimulation for the user.

### [BACKGROUND ART]

Recently, various sleep diseases and sleep disorders have been found based on physiological and physical signals output from a body of a subject during sleep, and based on this result, diagnosis and treatment directions for sleep problems have been established, and have been proposed for specific treatment methods such as drug therapy, cognitive behavioral correction, and surgical procedures.

For reference, brain waves are used as basic data to understand a progression and a structure of sleep, electrooculography (EOG) for measuring an eye movement is useful for determining sleep stages, and electromyography (EMG) has to be measured since muscle tension varies depending on the sleep stages. In particular, EMG for legs is measured to diagnose a sleep disorder called a periodic limb movement disorder, and EMG for a jaw is needed to identify bruxism.

In particular, a case of the bruxism includes clenching or grinding teeth by strong jaw movements other than chewing or swallowing movements, and usually the teeth are ground with a stronger force 2 to 10 times a force when chewing foods. The teeth are mostly ground during sleep, but in some cases, the teeth may be ground during the day.

It is known that approximately 10% of entire Korean population experiences the bruxism. The bruxism often occurs when a person is sensitive and in an unstable psychological, and stress is a cause of the bruxism. It is known that other causes not only include malocclusion in which the teeth do not fit well and drinking, but also include genetics, taking specific medications, and central nervous system disorders.

The bruxism may damage periodontal tissues since a strong force is partially applied to the teeth, and surfaces of the teeth may be worn down. Tissues around the teeth may be damaged, and the teeth may feel sensitive when a person eats cold foods. In severe cases, the teeth may even become loose.

The bruxism can be professionally treated at a hospital through psychological therapy, installing an occlusal stabilization device between upper and lower teeth, or medication.

Recently, there has been a demand for a device that can easily diagnose and prevent the bruxism during sleep at home. As one of technologies for methods for diagnosing the bruxism, Korean Patent Publication No. 10-1621500 discloses a device and a method for bruxism and teeth clenching analysis as follows. The device is mounted on a head of a subject to detect and output a tilt signal according to a change in a sleeping posture and an electromyography signal generated by the bruxism and teeth clenching of the subject. When a treatment signal is received from an analysis terminal, the device outputs a preset sleeping posture correction signal. However, the device and the method have limitations in accurately diagnosing symptoms of the bruxism and the teeth clenching, and do not show a significant awakening effect during sleep. In this regard, there is a problem in that a treatment effect for the bruxism and the teeth clenching is minimal.

Therefore, in order to solve the above-described problems, it is necessary to research a biofeedback-based bruxism alleviating system and a method thereof.

### [DETAILED DESCRIPTION OF INVENTION]

### [TECHNICAL PROBLEMS]

The present disclosure aims to provide a biofeedback-based bruxism alleviating system and a method thereof, which can more effectively detect occurrence of bruxism events of a user who falls asleep by collecting electromyography data on muscles around a face and a head of the user through a wearable device attached to skins of the user and determining whether the user falls asleep and whether the bruxism events occur based on the collected electromyography data.

In addition, the present disclosure aims to provide a biofeedback-based bruxism alleviating system and a method thereof, which can provide an optimized biofeedback stimulation signal for a user and can more effectively interfere with and alleviate bruxism symptoms by receiving an input of minimum intensity of a vibration stimulation to be provided through a wearable device from a user terminal and gradually increasing intensity of the stimulation from a minimum vibration stimulation input through the user terminal.

In addition, the present disclosure aims to provide a biofeedback-based bruxism alleviating system and a method thereof, which can collect and store specific data of each user by collecting and storing information on a sleep state and bruxism events of the user and can quickly calculate user-customized biofeedback data by constructing bruxism learning data, based on the collected data.

Aspects to be achieved by the present disclosure are not limited to the above-described aspects, and other aspects to be achieved by the present disclosure which are not described herein will be clearly understood by those skilled in the art to which the present disclosure belongs, from the description below.

### [TECHNICAL SOLUTION]

A biofeedback-based bruxism alleviating system according to one embodiment of the present disclosure includes a user recognition unit that receives a bruxism diagnosis request signal generated from at least one of a pre-designated user terminal and a wearable device attached to skins of a user, that extracts a unique number assigned to the wearable device included in the bruxism diagnosis request signal, and that calls up user information corresponding to the unique number from a pre-designated DB, a data collection unit that receives electromyography data collected from at least one sensor provided in the wearable device, a bruxism diagnosis unit that diagnoses occurrence of bruxism by determining whether the electromyography data collected from the data collection unit satisfies a preset condition, a biofeedback generation unit that applies a vibration stimulation to the skins of the user through a vibration motor provided in the wearable device, when the bruxism diagnosis unit determines the occurrence of the bruxism, and a user monitoring unit that monitors bruxism symptoms of the user, based on bruxism diagnosis data derived by the bruxism diagnosis unit and vibration stimulation data generated by the biofeedback generation unit.

In addition, the data collection unit may include a first sensor that collects first electromyography data for a preset period of time, and a second sensor provided at a preset interval from the first sensor to collect second electromyography data, and the bruxism diagnosis unit may include a sleep state determination unit that determines a sleep state of the user by comparing the first electromyography data collected through the first sensor with a preset first reference voltage V_th1, and a bruxism occurrence detection unit that determines occurrence of bruxism events of the user by comparing the second electromyography data collected through the second sensor with a preset second reference voltage V_th2, when the sleep state determination unit determines that the user falls asleep.

In addition, the bruxism occurrence detection unit may generate a window mask in a preset time range, may superimpose the window mask on the second electromyography data to determine the occurrence of the bruxism events, and may determine the occurrence of the bruxism events, based on a time during which the second electromyography data exceeds the preset second reference voltage V_th2 within the window mask, and the biofeedback generation unit may include a user stimulation providing unit that generates vibration stimulation having a first pattern at T_winend which is an end time of the window mask, and that provides the vibration stimulation having the first pattern to the user through the wearable device, and a user stimulation correction unit that corrects the vibration stimulation having the first pattern, when the bruxism events are detected through the bruxism diagnosis unit within a preset time range after the vibration stimulation having the first pattern is completed, and in the first pattern, an initial value of the vibration stimulation may be determined based on minimum stimulation intensity set from the user terminal, and intensity of the vibration stimulation may be corrected by each one level through the user stimulation correction unit.

In addition, the bruxism diagnosis unit may determine whether to use the electromyography data collected from the first sensor and the second sensor, based on a bruxism diagnosis mode input from the user terminal, and the bruxism diagnosis mode may include a first diagnosis mode for determining a sleep state of the user through the sleep state determination unit, and for determining the occurrence of the bruxism events of the user through the bruxism occurrence detection unit, when it is determined that the user falls asleep, and a second diagnosis mode for counting a preset sleep waiting time, automatically determining that the user falls asleep, when the sleep waiting time is exceeded, and determining the occurrence of the bruxism events of the user through the bruxism occurrence detection unit.

In addition, the user monitoring unit may include a user data generation unit that measures a daily sleep time of the user and generates daily user data including at least one of the number of bruxism events occurring during the daily sleep time, a duration of the bruxism, a degree of the bruxism, the number of vibration stimulation generation times, and maximum intensity of the vibration stimulation, a bruxism diagnosis learning unit that learns the number of the bruxism events occurring during the daily sleep time, the duration of the bruxism, and the maximum intensity of the vibration stimulation included in the daily user data through a preset artificial intelligence learning algorithm, and that optimizes a bruxism diagnosis reference of the bruxism diagnosis unit, based on result data derived through the artificial intelligence learning algorithm, and a user data transmission unit that transmits the daily user data generated by the user data generation unit to the user terminal, based on a request signal received from the user terminal.

### [EFFECT OF INVENTION]

According to the present disclosure, the electromyography data on muscles around a face and a head of the user is collected through the wearable device attached to skins of the user, and whether the user falls asleep and the occurrence of the bruxism events are determined, based on the collected electromyography data. In this manner, the present disclosure achieves an advantageous effect of more effectively detecting the occurrence of the bruxism events of the user who falls asleep.

In addition, an input of the minimum intensity of the vibration stimulation to be provided through the wearable device is received from the user terminal, and the intensity of the stimulation is gradually increased from the minimum vibration stimulation input through the user terminal. In this manner, the present disclosure provides an optimized biofeedback stimulation signal to the user, and achieves an advantageous effect of more effectively interfering with and alleviating the bruxism symptoms.

In addition, the sleep state of the user and bruxism event information are collected and stored. In this manner, the present disclosure achieves an advantageous effect of collecting user-specific data, constructing bruxism learning data based on the user-specific data, and quickly calculating user-customized biofeedback data.

### [BRIEF DESCRIPTION OF THE DRAWING]

FIGS. 1 and 2 are configuration diagrams of a biofeedback-based bruxism alleviating system according to one embodiment of the present disclosure.
FIG. 3 is a view for describing a data collection unit of the biofeedback-based bruxism alleviating system according to one embodiment of the present disclosure.
FIG. 4 is a view for describing a bruxism diagnosis unit of the biofeedback-based bruxism alleviating system according to one embodiment of the present disclosure.
FIGS. 5 to 8 are views for describing a biofeedback generation unit of the biofeedback-based bruxism alleviating system according to one embodiment of the present disclosure.
FIG. 9 is a view for describing a user monitoring unit of the biofeedback-based bruxism alleviating system according to one embodiment of the present disclosure.

### [BEST MODE FOR CARRYING OUT THE INVENTION]

Specific items including aspects to be achieved by the present disclosure as described above are included in embodiments and drawings which are described below. Advantages and features of the present disclosure and a method for achieving the advantages and the features will become clearer with reference to the embodiments described below in detail together with the accompanying drawings.

The scope of the present disclosure is not limited to the embodiments described below, and the present disclosure may be modified and implemented in various ways by those skilled in the art within a scope that does not depart from the technical concept of the present disclosure.

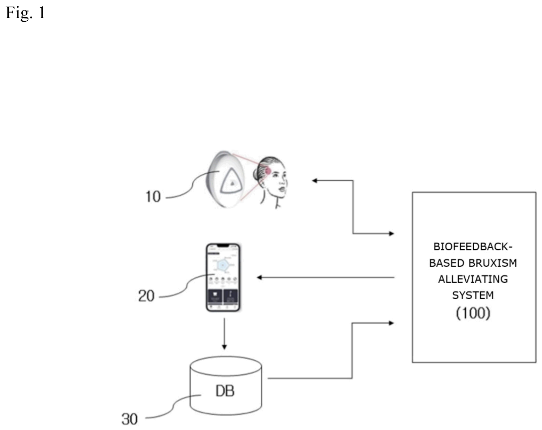

Hereinafter, the title of the invention as the present disclosure will be described in detail with reference to FIG. 1.

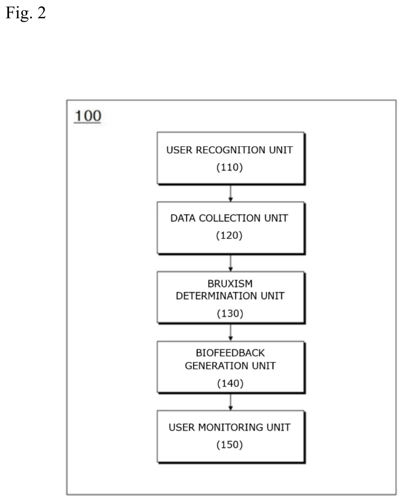

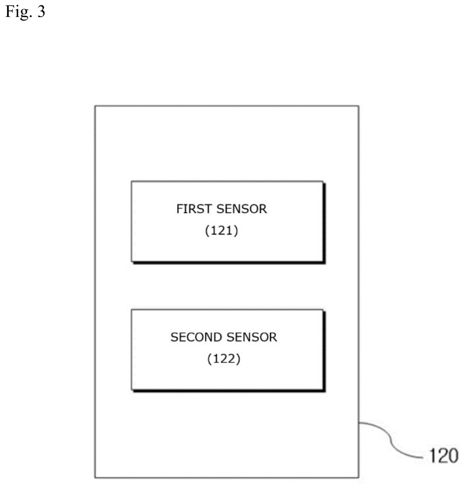

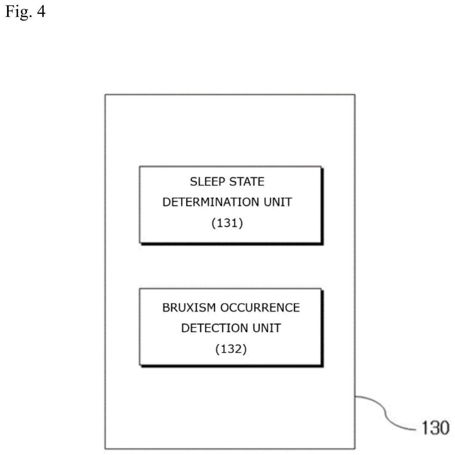

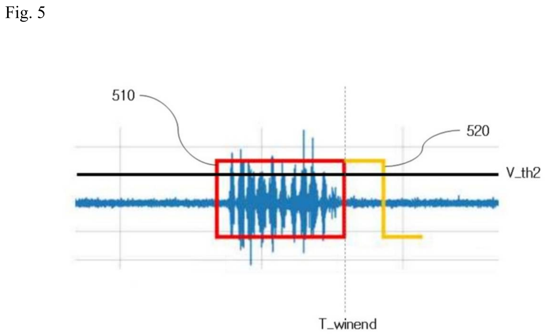

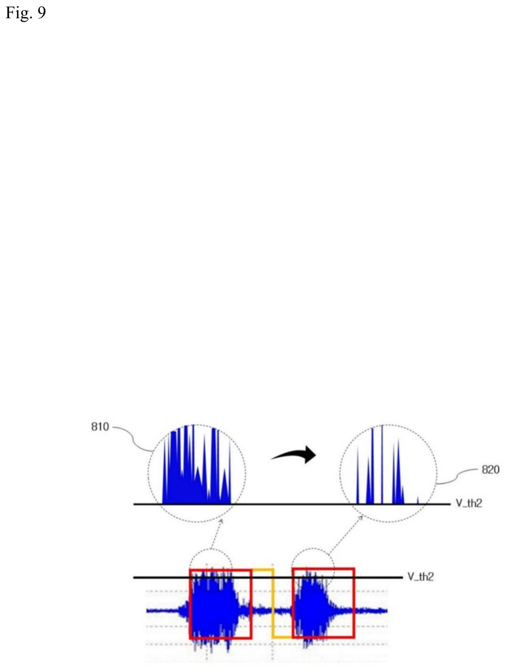

FIGS. 1 and 2 are configuration diagrams of a biofeedback-based bruxism alleviating system according to one embodiment of the present disclosure. FIG. 3 is a view for describing a data collection unit of the biofeedback-based bruxism alleviating system according to one embodiment of the present disclosure. FIG. 4 is a view for describing a bruxism diagnosis unit of the biofeedback-based bruxism alleviating system according to one embodiment of the present disclosure. FIGS 5 to 8 are views for describing a biofeedback generation unit of the biofeedback-based bruxism alleviating system according to one embodiment of the present disclosure. FIG. 9 is a view for describing a user monitoring unit of the biofeedback-based bruxism alleviating system according to one embodiment of the present disclosure.

### Embodiment 1

Referring to FIGS. 1 and 2, a biofeedback-based bruxism alleviating system 100 according to one embodiment of the present disclosure collects electromyography data required for diagnosing bruxism through a wearable device 10 attached to skins of a user or mounted on one side of a head of the user. The electromyography data collected through the wearable device 10 may be transmitted to the biofeedback-based bruxism alleviating system 100. The bruxism alleviating system 100 may analyze the electromyography data to diagnose bruxism symptoms of the user, and may generate a biofeedback signal for alleviating the bruxism symptoms in response to a result of the diagnosis. In this case, the biofeedback signal generated by the biofeedback-based bruxism alleviating system 100 may control a vibration motor provided in the wearable device 10 to provide a vibration stimulation to the user.

Accordingly, since the biofeedback such as the vibration stimulation is provided for the user, a function of an autonomic nervous system of the user may be regulated to induce the user to actively alleviate the bruxism symptoms.

In addition, data on the bruxism symptoms of the user which is generated through the biofeedback-based bruxism alleviating system 100 may be transmitted to the user terminal 20 linked with the wearable device 10. In addition, data transmitted to the user terminal 20 based on a request signal received from the user terminal 10 may be stored in a pre-designated DB 30.

More specifically, the biofeedback-based bruxism alleviating system 100 may include a user recognition unit 110, a data collection unit 120, a bruxism diagnosis unit 130, a biofeedback generation unit 140, and a user monitoring unit 150.

The user recognition unit 110 may receive a bruxism diagnosis request signal generated from at least one of the pre-designated user terminal 20 and the wearable device 10 attached to the skins of the user, may extract a unique number assigned to the wearable device 10 included in the bruxism diagnosis request signal, and may call up user information corresponding to the unique number from the pre-designated DB 30.

In addition, the data collection unit 120 may receive electromyography data collected from at least one sensor provided in the wearable device 10. The bruxism diagnosis unit 130 may determine whether the electromyography data collected from the data collection unit 120 satisfies a preset condition to diagnose occurrence of bruxism. The biofeedback generation unit 140 may apply a vibration stimulation to the skins of the user through a vibration motor provided in the wearable device 10, when the bruxism diagnosis unit 130 determines the occurrence of the bruxism. The user monitoring unit 150 may monitor the bruxism symptoms of the user, based on the bruxism diagnosis data derived by the bruxism diagnosis unit 130 and the vibration stimulation data generated by the biofeedback generation unit 140.

More specifically, as illustrated in FIG. 3, the data collection unit 120 may include a first sensor 121 that collects first electromyography data for a preset period of time and a second sensor 122 provided at a preset interval from the first sensor 121 to collect second electromyography data.

As an example, when the wearable device 10 is provided in a form of being attached to one side of the head of the user, the wearable device 10 may be attached near a temple of the user.

When the wearable device 10 is attached to one side of the temple of the user, electromyography data of a face of the user may be collected through the first sensor 121 and the second sensor 122 which are provided in the wearable device 10.

As an example, the first sensor 121 may collect an electromyography signal of an eye area of the user, and the second sensor 122 may collect an electromyography signal of a temporalis muscle of the user.

Meanwhile, referring to FIG. 4, the bruxism diagnosis unit 130 may include a sleep state determination unit 131 that determines a sleep state of the user by comparing the first electromyography data collected through the first sensor 121 with a preset first reference voltage V_th1, and a bruxism occurrence detection unit 132 that determines occurrence of bruxism events of the user by comparing the second electromyography data collected through the second sensor 122 with a preset second reference voltage V_th2, when the sleep state determination unit 131 determines that the user falls asleep.

In this case, the bruxism diagnosis unit 130 may determine whether to use data collected from the first sensor 121 and the second sensor 122, based on a bruxism diagnosis mode input to the user terminal 20. The bruxism diagnosis mode may include a first diagnosis mode for determining a sleep state of the user through the sleep state determination unit 131, and determining the occurrence of the bruxism events of the user through the bruxism occurrence detection unit 132, when it is determined that the user falls asleep, and a second diagnosis mode for counting a preset sleep waiting time, automatically determining that the user falls asleep when the sleep waiting time is exceeded, and determining the occurrence of the bruxism events of the user through the bruxism occurrence detection unit 132.

More specifically, when the bruxism diagnosis mode is set to the first diagnosis mode, the electromyography data may be collected by using both the first sensor 121 and the second sensor 122. While the first electromyography data is collected through the first sensor 121, the second sensor 122 may be switched to a power saving mode.

In addition, the sleep state determination unit 131 of the bruxism diagnosis unit 130 may analyze eye movements of the user by comparing the first electromyography data collected through the first sensor 121 with the first reference voltage V_thl1.

As an example, the sleep state determination unit 131 may generate eye movement data corresponding to a section in which the first electromyography data exceeds the first reference voltage V_th1, and may determine the sleep state of the user by comparing the eye movement data with eye movement learning data during sleep, which is stored in the DB 30.

When the sleep state determination unit 131 determines that the user falls asleep, the first sensor 121 may be switched to the power saving mode, and data collection through the first sensor 121 may be stopped.

In addition, when the bruxism diagnosis mode is set to the second diagnosis mode, the first sensor 121 is switched to the power saving mode, and the bruxism diagnosis unit 130 may determine the occurrence of bruxism symptoms of the user by using only the second electromyography data collected through the second sensor 121.

That is, in a case of the first diagnosis mode, the sleep time of the user may be accurately monitored, and in a process of determining the sleep state through the sleep state determination unit 131, the bruxism occurrence detection unit 132 is switched to the power saving mode. When the sleep state of the user is completely determined, the bruxism sleep state determination unit 131 is switched to the power saving mode through the bruxism occurrence detection unit 132. In this manner, battery power efficiency of the wearable device 10 may be improved, and in a case of the second diagnosis mode, a data processing process of the sleep state determination unit 131 may be omitted. Accordingly, a speed of a data calculation process may be improved.

Meanwhile, the bruxism occurrence detection unit 132 may collect second electromyography data on the movements of the temporalis muscle of the user through the second sensor 122, and may determine the occurrence of the bruxism events of the user by comparing the second electromyography data with the second reference voltage V_th2.

More specifically, as illustrated in FIG. 5, the bruxism occurrence detection unit 132 may generate a window mask 510 of a preset time range, may superimpose the window mask 510 on the second electromyography data to determine the occurrence of the bruxism events, and may determine the occurrence of the bruxism events, based on a time during which the second electromyography data exceeds the second reference voltage V_th2 within the window mask 510.

As an example, a time range of the window mask 510 is initially set to 2 seconds serving as a system setting value, and may be optimized through artificial intelligence learning of the user monitoring unit.

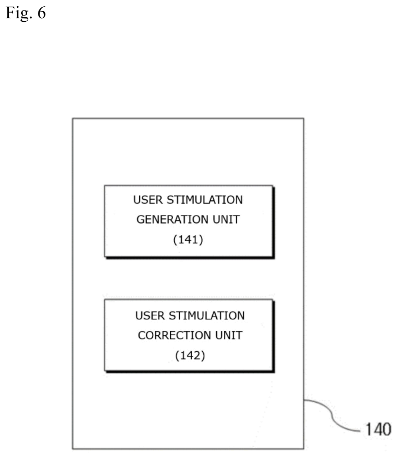

As illustrated in FIG. 6, the biofeedback generation unit 140 may include a user stimulation providing unit 141 that generates the vibration stimulation having a first pattern of at T_winend which is an end time of the window mask 510, when the bruxism occurrence detection unit 132 determines the occurrence of the bruxism events, and that provides the vibration stimulation having the first pattern to the user through the wearable device 10, and a user stimulation correction unit 142 that corrects the vibration stimulation having the first pattern, when the occurrence of the bruxism events is detected through the bruxism diagnosis unit 130 within a preset time range after the vibration stimulation having the first pattern is completed.

For example, when the bruxism diagnosis unit 130 determines the occurrence of the bruxism events within 5 seconds after the vibration stimulation having the first pattern is completed, the user stimulation correction unit 142 may correct the first pattern so that a stimulation having higher intensity than the vibration stimulation having the first pattern is provided to the user.

In this case, in the first pattern, an initial value of the vibration stimulation may be determined based on minimum stimulation intensity set from the user terminal 20, and intensity of the vibration stimulation may be corrected by each one level through the user stimulation correction unit 142.

As an example, when the intensity of the vibration stimulation is classified into levels 1 to 10 and the initial value of the vibration stimulation is input as the level 3 through the user terminal 20, the wearable device 10 may be started or restarted, and the initial value of the first pattern may be set to the level 3.

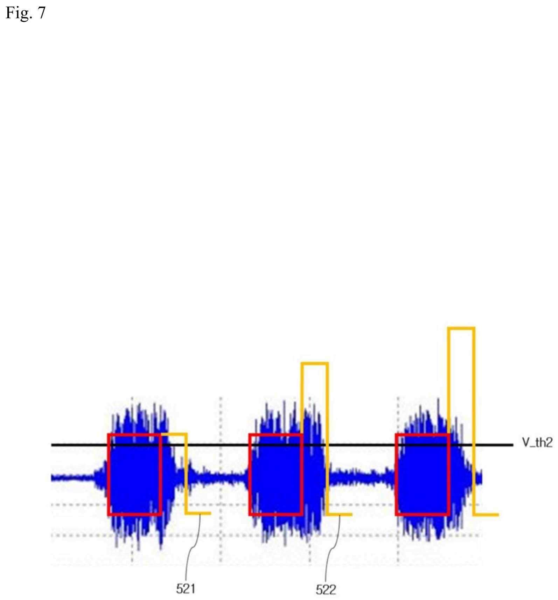

As illustrated in FIG. 7, when the bruxism diagnosis unit 130 determines the occurrence of the bruxism events, a vibration stimulation 521 having a first pattern set to the initial value of the level 3 is provided for the user through the biofeedback generation unit 140. When the vibration stimulation 521 having the first pattern is completed and the bruxism events occur again within 5 seconds, the bruxism symptoms of the user cannot be alleviated by the vibration stimulation at the level 3. Therefore, the user stimulation correction unit 142 may correct the vibration stimulation having the first pattern to the level 4.

Accordingly, with regard to the bruxism events occurring within 5 seconds after the vibration stimulation 521 having the first pattern is completed, the vibration stimulation 522 having the first pattern corrected to the level 4 may be provided for the user through the wearable device 10.

Similarly, when the bruxism events occur again within 5 seconds after the vibration stimulation 522 having the first pattern is completed, the bruxism symptoms of the user cannot be alleviated by the vibration stimulation at the level 4. Therefore, the user stimulation correction unit 142 may correct the vibration stimulation having the first pattern to the level 5.

In this case, the vibration stimulation having the first pattern may be corrected to a maximum level 10 of the vibration stimulation, and when the bruxism events occur again within 5 seconds after the vibration stimulation having the first pattern is corrected to the level 10, the user stimulation correction unit 142 may stop correcting the vibration stimulation having the first pattern, and may transmit a wake-up notification signal to the user terminal 20 to induce the user to wake up.

In addition, depending on settings of the user terminal, the wake-up notification signal may be transmitted to the user terminal after the first pattern is corrected to the level 10. After the correction is performed twice, the correction of the vibration stimulation having the first pattern may be stopped, and the wake-up notification signal may be immediately transmitted to the user terminal 20 to induce the user to wake up.

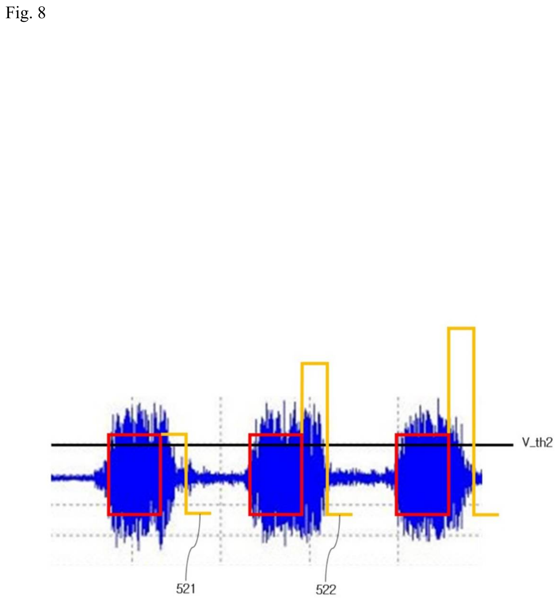

Meanwhile, as illustrated in FIG. 8, when the bruxism events do not occur within the preset time of 5 seconds after the vibration stimulation having the first pattern is applied to the user, the user stimulation correction unit 142 does not correct the vibration stimulation having the first pattern. Therefore, the user stimulation generation unit 141 may apply the vibration stimulation having the first pattern set to the initial value to the user through the wearable device 10 for the bruxism events occurring after the preset time of 5 seconds.

As another example, the user stimulation correction unit 142 may include a fixed pattern correction mode for increasing the vibration stimulation intensity of the first pattern by each one level and providing the same magnitude of the stimulation to the user for a preset time, and a variable pattern correction mode for correcting the vibration stimulation intensity of the first pattern to vary within the preset time.

For example, when the minimum stimulation intensity at the level 3 is input from the user terminal 20 and the maximum stimulation intensity at the level 6 is input, the fixed pattern correction mode may be applied. In this manner, the vibration stimulation having the first pattern may be corrected and provided for the user such that the vibration stimulation having the first pattern is variable for a preset unit time (for example, 0.5 seconds) set in advance to any value between the level 3 and the level 6 within the preset time.

Therefore, the user with the vibration stimulation that varies from a weak stimulation to a strong stimulation rather than the vibration stimulation having a fixed magnitude. In this manner, more effective regulation of the autonomic nervous system may be induced.

Meanwhile, the bruxism diagnosis unit 130 may determine the occurrence of the bruxism, based on the second electromyography data collected through the data collection unit 120 while the vibration stimulation generated through the biofeedback generation unit 140 is applied to the user.

Therefore, all of the bruxism-related electromyography data of the user may be collected without data omission, and more accurate bruxism diagnosis and customized biofeedback may be provided.

Meanwhile, referring to FIG. 9, the user monitoring unit 150 may include a user data generation unit 151 that measures a daily sleep time of the user, and that generates daily user data including at least one of the number of the bruxism events occurring during the daily sleep time, a degree of the bruxism, a duration of the bruxism, the number of vibration stimulation generation times, and the maximum intensity of the vibration stimulation, a bruxism diagnosis learning unit 152 that learns the number of the bruxism events occurring during the daily sleep time, the duration of the bruxism, and the maximum intensity of the vibration stimulation included in the daily user data through a preset artificial intelligence learning algorithm, and that optimizes a bruxism diagnosis reference of the bruxism diagnosis unit, based on result data derived through the artificial intelligence learning algorithm, and a user data transmission unit 153 that transmits the daily user data generated by the user data generation unit 151 to the user terminal 20, based on a request signal received from the user terminal 20.

As an example, the bruxism diagnosis learning unit 152 may optimize a time range of the window mask by using any one of a mean, a median, and a mode of the bruxism duration included in the daily user data.

In addition, a degree of the bruxism of the user may be calculated, based on the second electromyography data, and a magnitude of the second reference voltage V_th2 may be corrected, based on the degree of the bruxism.

Accordingly, when the degree of the bruxism is classified into 'strong', 'average' and 'weak', and the degree of the bruxism of the user is calculated as the 'weak', the magnitude of the second reference voltage V_th2 may be reduced to induce the bruxism symptoms to be alleviated through the bruxism diagnosis and the biofeedback which are optimized for the user.

As another example, the bruxism diagnosis learning unit 152 may verify the bruxism diagnosis of the bruxism diagnosis unit 140. The bruxism diagnosis learning unit 152 may verify the bruxism diagnosis of the bruxism diagnosis unit 140 by using a correct classification dataset for determining bruxism as bruxism and determining non-bruxism as non-bruxism and an incorrect classification dataset for determining bruxism as non-bruxism and determining non-bruxism as bruxism. In this manner, accuracy of the bruxism diagnosis of the bruxism diagnosis unit 140 may be improved.

According to the present disclosure as described above, there are provided the biofeedback-based bruxism alleviating system and the method thereof, which can more effectively detect the occurrence of the bruxism events of the user who falls asleep by collecting the electromyography data on muscles around the face and the head of the user through the wearable device attached to the skins of the user, and determining the occurrence of the bruxism events, based on the collected electromyography data.

In addition, there are provided the biofeedback-based bruxism alleviating system and the method thereof, which can provide the optimized biofeedback stimulation signal for the user and can more effectively interfere with and alleviate the bruxism symptoms by receiving an input of the minimum intensity of the vibration stimulation to be provided through a wearable device from the user terminal and gradually increasing the intensity of the stimulation from minimum vibration stimulation input through the user terminal.

In addition, there are provided the biofeedback-based bruxism alleviating system and the method thereof, which can collect and store specific data of each user by collecting and storing information on the sleep state and the bruxism events of the user and can quickly calculate user-customized biofeedback data by constructing bruxism learning data, based on the collected data.

In addition, according to an embodiment of the present disclosure, there is provided a method for controlling the biofeedback-based bruxism alleviating system. The method may be recorded on a computer-readable medium including program commands for performing various computer-implemented operations. The computer-readable medium may include program commands, data files, data structures, and the like alone or in combination. The program commands may be specially designed and configured for the present disclosure, or may be those known to and available to those skilled in the art of computer software. Examples of the computer-readable recording medium include magnetic media such as a hard disk, a floppy disk, and a magnetic tape, optical media such as a CD-ROM and a DVD, magneto-optical media such as a floptical disk, and hardware devices specially configured to store and execute program commands, such as a ROM, a RAM, and a flash memory. Examples of the program commands include not only machine language codes generated by a compiler, but also high-level language codes which can be executed by a computer by using an interpreter or the like.

Although the embodiments of the present disclosure have been described with limited embodiments and drawings, the embodiments of the present disclosure are not limited to the embodiments described above. Various corrections and modifications can be made from this description by those skilled in the art to which the present disclosure belongs. Accordingly, the embodiments of the present disclosure should be understood only by the scope of the appended claims, and all equivalent or equivalent variations thereof will fall within the scope of the present disclosure.

### [DESCRIPTION OF REFERENCE NUMERALS]

10: wearable device
20: user terminal
30: DB
110: user recognition unit
120: data collection unit
121: first sensor
122: second sensor
130: bruxism diagnosis unit
131: sleep state determination unit
132 bruxism occurrence detection unit
140 biofeedback generation unit
141 user stimulation generation unit
142 user stimulation correction unit
150 user monitoring unit
151: user data generation unit
152: bruxism diagnosis learning unit
153: user data transmission unit

## Claims

1. A biofeedback-based bruxism alleviating system comprising:
a user recognition unit that receives a bruxism diagnosis request signal generated from at least one of a predetermined user terminal and a wearable device attached to skins of a user, that extracts a unique number assigned to the wearable device included in the bruxism diagnosis request signal, and that calls up user information corresponding to the unique number from a predetermined DB;
a data collection unit that receives electromyography data collected from at least one sensor provided in the wearable device;
a bruxism diagnosis unit that diagnoses occurrence of bruxism by determining whether the electromyography data collected from the data collection unit satisfies a preset condition;
a biofeedback generation unit that applies a vibration stimulation to the skins of the user through a vibration motor provided in the wearable device, when the bruxism diagnosis unit determines the occurrence of the bruxism; and
a user monitoring unit that monitors bruxism symptoms of the user, based on bruxism diagnosis data derived by the bruxism diagnosis unit and vibration stimulation data generated by the biofeedback generation unit.

2. The biofeedback-based bruxism alleviating system of claim 1, wherein the data collection unit includes a first sensor that collects first electromyography data for a preset period of time, and a second sensor provided at a preset interval from the first sensor to collect second electromyography data, and the bruxism diagnosis unit includes a sleep state determination unit that determines a sleep state of the user by comparing the first electromyography data collected through the first sensor with a preset first reference voltage V_th1, and a bruxism occurrence detection unit that determines occurrence of bruxism events of the user by comparing the second electromyography data collected through the second sensor with a preset second reference voltage V_th2, when the sleep state determination unit determines that the user falls asleep.

3. The biofeedback-based bruxism alleviating system of claim 2, wherein the bruxism occurrence detection unit generates a window mask in a preset time range, superimposes the window mask on the second electromyography data to determine the occurrence of the bruxism events, and determines the occurrence of the bruxism events, based on a time during which the second electromyography data exceeds the preset second reference voltage V_th2 within the window mask, and the biofeedback generation unit includes a user stimulation providing unit that generates vibration stimulation having a first pattern at T_winend which is an end time of the window mask, and that provides the vibration stimulation having the first pattern to the user through the wearable device, and a user stimulation correction unit that corrects the vibration stimulation having the first pattern, when the bruxism events are detected through the bruxism diagnosis unit within a preset time range after the vibration stimulation having the first pattern is completed, and in the first pattern, an initial value of the vibration stimulation is determined based on minimum stimulation intensity set from the user terminal, and intensity of the vibration stimulation is corrected by each one level through the user stimulation correction unit.

4. The biofeedback-based bruxism alleviating system of claim 2, wherein the bruxism diagnosis unit determines whether to use the electromyography data collected from the first sensor and the second sensor, based on a bruxism diagnosis mode input from the user terminal, and the bruxism diagnosis mode includes a first diagnosis mode for determining a sleep state of the user through the sleep state determination unit, and for determining the occurrence of the bruxism events of the user through the bruxism occurrence detection unit, when it is determined that the user falls asleep, and a second diagnosis mode for counting a preset sleep waiting time, automatically determining that the user falls asleep, when the sleep waiting time is exceeded, and determining the occurrence of the bruxism events of the user through the bruxism occurrence detection unit.

5. The biofeedback-based bruxism alleviating system of claim 1, wherein the user monitoring unit includes a user data generation unit that measures a daily sleep time of the user and generates daily user data including at least one of the number of bruxism events occurring during the daily sleep time, a duration of the bruxism, a degree of the bruxism, the number of vibration stimulation generation times, and maximum intensity of the vibration stimulation, a bruxism diagnosis learning unit that learns the number of the bruxism events occurring during the daily sleep time, the duration of the bruxism, and the maximum intensity of the vibration stimulation included in the daily user data through a preset artificial intelligence learning algorithm, and that optimizes a bruxism diagnosis reference of the bruxism diagnosis unit, based on result data derived through the artificial intelligence learning algorithm, and a user data transmission unit that transmits the daily user data generated by the user data generation unit to the user terminal, based on a request signal received from the user terminal.
